(19)

Europäisches
Patentamt
European
Patent Office
Office européen
des brevets

(11)  **EP 4 180 528 A1**

(12)  # EUROPEAN PATENT APPLICATION
published in accordance with Art. 153(4) EPC

(43) Date of publication:
**17.05.2023   Bulletin 2023/20**

(21) Application number: **21842930.6**

(22) Date of filing: **12.07.2021**

(51) International Patent Classification (IPC):
**C12P 1/00** (2006.01)        **A23L 5/00** (2016.01)
**A23L 29/00** (2016.01)       **A23L 33/185** (2016.01)
**C12N 9/20** (2006.01)        **A23C 11/10** (2021.01)
**A23L 11/60** (2021.01)       **A23L 11/65** (2021.01)
**A23L 11/00** (2021.01)

(52) Cooperative Patent Classification (CPC):
**A23C 11/10; A23L 5/00; A23L 11/00; A23L 11/60;
A23L 11/65; A23L 29/00; A23L 33/185; C12P 1/00;
C12N 9/20**

(86) International application number:
**PCT/JP2021/026185**

(87) International publication number:
**WO 2022/014542 (20.01.2022 Gazette 2022/03)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **13.07.2020   JP 2020120050**

(71) Applicants:
• **Amano Enzyme Inc.
Nagoya-shi
Aichi 460-8630 (JP)**

• **Amano Enzyme Asia Pacific Co., Ltd.
Pathum Thani 12120 (TH)**

(72) Inventor: **NAKNUKOOL, Supaporn
Pathum Thani 12120 (TH)**

(74) Representative: **Müller-Boré & Partner
Patentanwälte PartG mbB
Friedenheimer Brücke 21
80639 München (DE)**

(54)  **METHOD FOR PRODUCING VEGETABLE PROTEIN FOOD**

(57)     The present invention addresses the problem of providing a novel production method of a plant protein food. This method comprises treating a starting plant protein material with both of a lipase and a protein deamidase (for example, protein glutaminase) to thereby improve the flavor of a plant protein food.

**EP 4 180 528 A1**

**Description**

TECHNICAL FIELD

The present invention relates to a novel method for producing a plant protein food using an enzyme. For example, the present invention is useful for the production of a fermented food (particularly, plant yogurt).

BACKGROUND ART

Against the background of allergy problems, an increase in the number of vegetarians, religious reasons, and the like, proteins of plant origins such as soybeans, cereals, and nuts have come into widespread use as alternative raw materials for foods and beverages using a milk protein source derived from animals, typified by milk.

When milk protein raw materials are replaced with plant-derived protein raw materials, types and functionalities of proteins and fats and oils contained in the plant-derived protein raw materials, or components constituting smell and taste are different, and thus improvement in texture, taste, smell, and the like is required. For example, there is known a method for obtaining an odorless and palatable protein by causing a lipase to act on a soybean protein and then removing the resulting hydrolyzate (Patent Document 1).

On the other hand, in recent years, interest in health is increasing, and the demand for a fermented food such as yogurt is increasing. There are various findings on the use of enzymes in fermented foods, and for example, there are known a method of enhancing a cheese flavor by using a lactase and a lipase, a method of smoothing the texture of yogurt by using a transglutaminase, a method of improving the texture and flavor of yogurt by using a protein deamidase, and a method of improving the flavor of yogurt by using a lipase (see, for example, Patent Documents 2, 3, 4, and 5). However, these documents do not refer to improving the flavor and properties of plant protein foods such as soy milk and soy milk yogurt. As a method of improving the flavor of soy milk yogurt, a method using fumaric acid is known (see, for example, Patent Document 6).

PRIOR ART DOCUMENTS

PATENT DOCUMENTS

Patent Document 1: Japanese Patent Laid-open Publication No. H06-030710
Patent Document 2: Japanese Patent Laid-open Publication No. 2011-525356
Patent Document 3: Japanese Patent Laid-open Publication No. H06-197688
Patent Document 4: WO 2006/075772 A
Patent Document 5: Japanese Patent Laid-open Publication No. 2003-250482
Patent Document 6: Japanese Patent Laid-open Publication No. H07-031371

SUMMARY OF THE INVENTION

PROBLEMS TO BE SOLVED BY THE INVENTION

An object of the present invention is to provide a method for producing a plant protein food having at least excellent flavor, preferably also having excellent properties in addition to the flavor.

MEANS FOR SOLVING THE PROBLEM

The present inventor has conducted intensive studies in order to solve the above problems, and as a result, has found that, when a lipase and a protein deamidase are caused to act on a protein raw material in production of a plant protein food, an effect of improving flavor (for example, an effect of imparting a clear fermentation smell to a fermented food) is obtained, and in some cases, an effect of improving properties (for example, an effect of improving smoothness) is obtained in addition to the effect of improving flavor, thereby completing the present invention described below.

[1] A method for producing a plant protein food, including causing a lipase and a protein deamidase to act on a plant protein raw material containing a protein and a fat and oil.
[2] The method for producing a plant protein food described in [1], in which the plant protein raw material is a soybean-derived raw material, an oat-derived raw material, an almond-derived raw material, or a coconut-derived raw material.

[3] The method for producing a plant protein food described in [1] or [2], in which the method includes steps below:

(1) a step of preparing a plant protein raw material containing a protein and a fat and oil; and
(2) a step of treating the prepared plant protein raw material with a lipase and a protein deamidase.

[4] The method for producing a plant protein food described in [3], in which the plant protein food is a fermented food, and the method includes a step below after the step (2):
(3) a step of fermenting with a microorganism.
[5] The method for producing a plant protein food described in [4], in which the fermented food is a lactic acid fermented food, and the microorganism in the step (3) is lactobacillus.
[6] The method for producing a plant protein food described in any one of [1] to [5], in which the lipase is a Candida cylindracea-derived lipase.
[7] The method for producing a plant protein food described in any one of [1] to [6], in which the protein deamidase is an enzyme acting on a glutamine residue in a protein.
[8] The method for producing a plant protein food described in [7], in which the protein deamidase is protein glutaminase.
[9] A plant protein improver containing a lipase and a protein deamidase.
[10] The plant protein improver described in [9], in which the lipase is a Candida cylindracea-derived lipase.
[11] The plant protein improver described in [9] or [10], in which the protein deamidase is an enzyme acting on a glutamine residue in a protein.
[12] The plant protein improver described in [11], in which the protein deamidase is protein glutaminase.
[13] The plant protein improver described in any one of [9] to [11], in which the plant protein improver is used as a fermentation smell enhancer for a plant fermented food.
[14] The plant protein improver described in any one of [9] to [12], in which the plant protein improver is used as a smoothness improver for a plant protein food.

EMBODIMENTS OF THE INVENTION

1. Method for Producing Plant Protein Food

A method for producing a plant protein food of the present invention includes causing a lipase and a protein deamidase to act on a plant protein raw material containing a protein and a fat and oil.

1-1. Plant Protein Food

The plant protein food obtained by the production method of the present invention is not particularly limited. Examples of the plant protein food include plant fermented foods, and those to which at least a flavor (particularly, distinct fermentation smell) improved by the production method of the present invention is imparted, preferably those to which an improved property (particularly, smoothness) is imparted in addition to the improved flavor can be exemplified. Specific examples of the plant fermented food include a lactic acid fermented plant food, more specifically, an alternative milk fermented food, further specifically, alternative yogurt (also called plant yogurt; solid content other than the fat and oil: 8.0 wt% or more), an alternative dairy product lactobacillus beverage (solid content other than the fat and oil: 3.0 wt% or more and less than 8.0 wt%), an alternative lactobacillus beverage (solid content other than the fat and oil: less than 3.0 wt%), and an alternative cheese (also called plant cheese; solidified products of alternative milk fermented foods). Examples of the plant protein food include a plant protein beverage (for example, alternative milk (also called plant milk)), bean curd, a meat alternative prepared from a plant raw material, alternative dairy products prepared from plant raw materials (for example, plant milk processed products such as an alternative milk fermented food), and those to which at least a flavor (particularly, distinct fermentation smell) improved by the production method of the present invention is imparted, preferably those to which an improved property (particularly, smoothness) is imparted in addition to the improved flavor can also be exemplified. Examples of the alternative milk fermented food are as described above. Among the above examples, a preferable plant protein food in the production method of the present invention is a lactic acid fermented plant food, and among the lactic acid fermented plant foods, an alternative milk fermented food is preferable and alternative yogurt (plant yogurt) is particularly preferable.

1-2. Plant Protein Raw Material

There are no particular restrictions on the origin, properties, and the like of the plant protein raw material as a raw material for the plant protein food.

For example, the origin of the protein contained in the plant protein raw material is not particularly limited as long as it is a plant, and specific examples thereof include beans such as soy beans, green peas, lentils, chickpeas, black beans, horse beans, mung beans, lupine beans, and kidney beans; cereals such as wheat, barley, oats, rice, rye, buckwheat, Japanese millet, foxtail millet, and teff; nuts such as almonds, coconuts, peanuts, cashew nuts, hazelnuts, pecan nuts, macadamia nuts, pistachio, walnuts, brazil nuts, pilinuts, chestnuts, sesame, and pine nuts; and seeds such as hemp, chia seeds, quinoa, amaranthus, canary seeds, and linseed. As the protein contained in the plant protein raw material, it is also possible to use a chemically partially decomposed protein of the above-described protein by an acid, an alkali, or the like, an enzymatically partially decomposed protein by a protease or the like, a chemically modified protein by various reagents, a synthetic peptide, or the like. In the present invention, as these proteins, a single plant-derived protein may be used, or two or more kinds of plant-derived proteins may be used in combination. Among these proteins, proteins derived from beans, cereals, and nuts are preferably exemplified, and proteins derived from soybeans, almonds, oats, and coconuts are more preferably exemplified.

The plant protein raw material contains a fat and oil in addition to the protein. The origin of the fat and oil is not particularly limited as long as it is a plant. Specific examples of plants as the origin of the fat and oil can be selected from the specific examples of plants mentioned as the origin of the protein. The plant as the origin of the fat and oil may be the same as or different from the plant as the origin of the protein, but is preferably the same as the plant as the origin of the protein.

The property of the plant protein raw material to be subjected to an enzyme treatment is not particularly limited, but examples thereof include a liquid form, a slurry form, and a paste form, and examples thereof preferably include a liquid form, a slurry form, and a paste form which form an emulsion with the fat and oil.

In the present invention, a preferable example of the plant protein raw material to be subjected to the enzyme treatment is plant milk, a more preferable example thereof is plant milk using beans as a raw material, plant milk using cereals as a raw material, and plant milk using nuts as a raw material, and a further preferable example thereof is soy milk, almond milk, oat milk, and coconut milk.

The method for preparing the plant protein raw material is not particularly limited. For example, the plant protein raw material can be prepared by the following preparation method. As the preparation method, a method capable of preparing a mixture containing the protein and the fat and oil, preferably an emulsion in which the fat and oil are dispersed in water containing the protein can be appropriately selected by those skilled in the art from known methods.

Specific examples of the method for preparing the plant protein raw material include (i) a method in which a dried powder of a plant as the origin of the protein and the fat and oil is dispersed in water; (ii) a method in which a plant as the origin of the protein and the fat and oil is crushed or ground in water, dispersed, and filtered as necessary; (iii) a mixture obtained by, for example, removing at least a part of components other than the protein and the fat and oil derived from the plant from the liquid obtained by the above method (i) or (ii) to increase the contents of the protein and the fat and oil derived from the plant; (iv) a liquid obtained by further diluting the liquid obtained by the above method (i) or (ii); and (v) a liquid obtained by dissolving and/or dispersing a solid such as a powder prepared by drying any of the liquids of the above (i) to (iv) in water. The plant protein raw materials prepared by these methods (i) to (iv) can be used as plant milk when the plant protein raw materials are liquid.

Other specific examples of the method for preparing the plant protein raw material include a method in which a protein purified from any of the above-described plants and a fat and oil purified from any of the above-described plants are mixed in, for example, water or the like.

In addition to the above, particularly, when plant milk is used as the plant protein raw material, commercially available plant milk can also be used as it is, by being diluted with water, or by being concentrated by removing moisture.

The protein content and the fat-and-oil content in the plant protein raw material, and the ratio thereof are not particularly limited, and can be appropriately determined according to the properties and conditions desired for the plant protein food, the type of the plant as the origin of the protein and fat-and-oil, and the like. For example, the protein content in the plant protein raw material is, for example, 0.1 to 30 wt%, preferably 0.3 to 20 wt% and 0.5 to 15 wt%, and further preferably 0.9 to 11 wt%. The fat-and-oil content in the plant protein raw material is, for example, 0.5 to 30 wt%, preferably 1 to 25 wt% and 2 to 20 wt%, and more preferably 2.5 to 15 wt%. In the plant protein raw material, as for the content ratio of the protein and the fat and oil, the fat-and-oil content is, for example, 0.05 to 50 parts by weight, and 0.1 to 30 parts by weight, preferably 0.3 to 20 parts by weight and 0.5 to 10 parts by weight, and more preferably 0.7 to 7 parts by weight, with respect to 1 part by weight of the protein.

Salts, saccharides, proteins other than the plant protein, fragrances, moisturizers, colorants, and the like may be added to the plant protein raw material as necessary.

1-3. Lipase and Protein Amidase

The lipase described in the present invention is an enzyme that releases a fatty acid from the fat and oil by a hydrolysis reaction. The type, origin, and the like of the lipase usable in the present invention are not particularly limited. The origin thereof is, for example, of animal, plant, or microbial origin. For example, a lipase derived from the genus of Rhizopus, Penicillium, Burkholderia, Aspergillus, Candida, Pichia, Chromobacterium, Pseudomonas, Mucor, Thermomyces, or Geotrichum can be used. Preferably, a lipase derived from the genus of Candida is employed. An example of the lipase derived from the genus of Candida is a lipase produced by Candida cylindracea (specifically, Lipase AY (Amano Enzyme Inc.)).

The protein deamidase described in the present invention refers to an enzyme that deamidates an amide group of a glutamine residue and an asparagine residue in a protein. The type, origin, and the like of the protein deamidase that can be used in the present invention are not particularly limited. The origin thereof is, for example, of animal, plant, or microbial origin. As an example of an enzyme that deamides the glutamine residue in the protein, Chryseobacterium proteolyticum-derived protein glutaminase (Eur J Biochem, 268 (5), 1410, 2001, Protein-glutaminase From Chryseobacterium Proteolyticum, an Enzyme That Deamidates Glutaminyl Residues in Proteins. Purification, Characterization and Gene Cloning, S Yamaguchi 1, D J Jeenes, D B Archer or Front Microbiol, 9, 1975, 2018, Complete Genome Sequence and Characterization of a Protein-Glutaminase Producing Strain, Chryseobacterium proteolyticum QSH1265, Ruidan Qu, Xiaoyu Zhu, Min Tian, Yingjie Liu, Wenjuan Yan, Jian Ye, Hongliang Gao, Jing Huang) is well known, but the enzyme is not limited thereto. An enzyme that deamidates the asparagine residue in the protein is disclosed in, for example, WO 2015/133590 A, but is not limited thereto. The protein deamidase described in the present invention also includes an enzyme that deiminizes an arginine residue. As the enzyme that deiminizes the arginine residue, for example, Fusarium graminearum-derived arginine deiminase is known.

In general, when glutamine and asparagine residues in the protein are deamidated to generate a carboxyl group, the negative charge of the protein increases, and as a result, the isoelectric point decreases and the hydration force increases. An increase in electrostatic repulsive force leads to a decrease in interaction between proteins, that is, a decrease in associative properties. These changes greatly increase the solubility and water dispersibility of the protein. The increase in negative charge of the protein loosens the folding of the protein, changes the higher-order structure, and exposes a hydrophobic region that has been buried in the inside of the molecule to the molecular surface. Therefore, the deamidated protein has amphiphilicity and becomes an ideal surfactant, and the emulsification power, emulsification stability, foamability, and foam stability of the protein are greatly improved. In this way, the deamidation of the protein leads to improvement in various functional properties of the protein, and the use of the protein is dramatically increased (for example, Molecular Approaches to Improving Food Quality and Safety, D. Chatnagar and T. E. Cleveland, eds., Van Nostrand Reinhold, New York, 1992, p. 37). Also when the arginine residue in the protein is deiminized, the hydrophobicity of the protein is increased to change the higher-order structure of the protein.

The method of acquiring the lipase and the protein deamidase that can be used in the present invention is also not particularly limited. For example, when the lipase and/or the protein deamidase is an enzyme derived from a microorganism, the lipase and/or the protein deamidase may be accumulated in either the inside or outside of the microorganism. Not only a lipase and/or a protein deamidase existing in nature but also a lipase and/or a protein deamidase produced by a genetic engineering technique or a cell engineering technique may be used. An enzyme protein modified by a protein engineering technique may be used. As the lipase and the protein deamidase (for example, protein glutaminase), it is desired to use a purified enzyme having a high purity, but the purity is not limited as long as a desired reaction is possible. An enzyme preparation may be used as the lipase and the protein deamidase, and in this case, various salts, saccharides, proteins, lipids, surfactants, and the like may be added as an enzyme stabilizer to the enzyme preparation.

The amount of the enzyme used is not particularly limited. Usually, the amount of the enzyme is 0.01 to 2000 U, preferably 0.1 to 1000 U, more preferably 1 to 500 U, and further preferably 10 to 400 U in terms of an activity value of the lipase by a kit method, and the amount of the protein deamidase is 0.1 to 10000 U, preferably 1 to 1000 U, more preferably 2 to 100 U, further preferably 3 to 50 U, and even more preferably 4 to 20 U, per 1 g of the protein.

When the plant protein raw material is obtained by using beans (preferably, soybeans) as a raw material and is more preferably soy milk, the amount of the lipase used per 1 g of the protein is, for example, 1 to 500 U, preferably 5 to 300 U, and more preferably 10 to 230 U in terms of an activity value by a kit method, and the amount of the protein amidase used per 1 g of the protein is, for example, 1 to 40 U, preferably 2 to 20 U, and more preferably 4 to 16 U.

When the plant raw material is obtained by using cereals (preferably, oats) as a raw material and is more preferably oat milk, the amount of the lipase used per 1 g of the protein is, for example, 50 to 600 U, preferably 100 to 500 U, more preferably 200 to 400 U, and even more preferably 250 to 350 U in terms of an activity value by a kit method, and the amount of the protein deamidase used per 1 g of the protein is, for example, 0.5 to 15 U, preferably 2 to 10 U, and more preferably 4 to 7 U.

When the plant raw material is obtained by using nuts (preferably, almonds) as a raw material and is more preferably almond milk, the amount of the lipase used per 1 g of the protein is, for example, 100 to 700 U, preferably 200 to 600 U, more preferably 300 to 500 U, and even more preferably 350 to 400 U in terms of an activity value by a kit method, and the amount of the protein deamidase used per 1 g of the protein is, for example, 0.5 to 15 U, preferably 2 to 10 U, and more preferably 4 to 7 U.

When the plant raw material is obtained by using nuts (preferably, coconuts) as a raw material and is more preferably coconut milk, the amount of the lipase used per 1 g of the protein is, for example, 1 to 100 U, preferably 5 to 50 U, more preferably 10 to 25 U, and further preferably 15 to 20 U in terms of an activity value by a kit method, and the amount of the protein deamidase used per 1 g of the protein is, for example, 5 to 40 U, preferably 10 to 20 U, and more preferably 12 to 17 U.

1-4. Operation Procedure and Others

The reaction time, temperature, pH of the reaction solution, and the like for causing the lipase and the protein deamidase to act on the plant protein raw material are also not particularly limited. The reaction temperature is, for example, 5 to 80°C, preferably 20 to 70°C, and more preferably 30 to 60°C. The pH of the reaction solution is, for example, 2 to 10 and preferably 4 to 8. The reaction time is, for example, 10 seconds to 48 hours and preferably 10 minutes to 24 hours. The physical properties of the plant protein raw material are changed depending on the above reaction conditions, at least flavor is improved, and the property is improved in addition to the improvement in flavor in some cases. These reaction conditions are appropriately selected according to a target plant protein food. The optimum reaction conditions may be determined through a preliminary experiment.

In the method for producing a plant protein food in the present invention, the lipase and the protein deamidase are caused to act on the plant protein raw material. In other words, the plant protein raw material is treated with the lipase and the protein deamidase. The order of action of the enzymes (that is, the order of the treatment with the lipase and the treatment with the protein deamidase) is not particularly limited, but it is preferable to perform the treatments simultaneously for the purpose of improving work efficiency and the like. It has been confirmed that an excellent effect is obtained by simultaneously performing the treatments with the lipase and the protein deamidase (see Examples described below).

By using the production method of the present invention, a food having at least improved flavor, preferably a food having improved properties in addition to the flavor can be produced. An embodiment of the method for producing a plant protein food of the present invention includes the following steps (1) and (2). An enzyme deactivation step may be added after the step (2).

(1) Step of preparing a plant protein raw material containing a protein and a fat and oil
(2) Step of treating the prepared plant protein raw material with a lipase and a protein deamidase

When a plant protein food to be produced is a fermented food, a fermentation step (3) below is performed after the above step (2).

(3) Step of fermenting with a microorganism

In the fermentation step, various microorganisms (mold, yeasts, and bacteria) are used. Microorganisms suitable for a plant protein food to be produced will be used. For example, when a lactic acid fermented food is produced as the plant protein food, lactobacillus may be employed. That is, in the case of this example, the "step of fermenting with lactobacillus" is performed.

2. Plant Protein Food

The present invention also provides a plant protein food obtained by the above-described production method. The plant protein food of the present invention is not limited as described above, examples thereof include plant fermented foods, and those to which at least a flavor (particularly, distinct fermentation smell) improved by the production method of the present invention is imparted, preferably those to which an improved property (particularly, smoothness) is imparted in addition to the improved flavor can be exemplified. Specific examples of the plant fermented food include a lactic acid fermented plant food, more specifically, an alternative milk fermented food, further specifically, alternative yogurt (also called plant yogurt; solid content other than the fat and oil: 8.0 wt% or more), an alternative dairy product lactobacillus beverage (solid content other than the fat and oil: 3.0 wt% or more and less than 8.0 wt%), an alternative lactobacillus beverage (solid content other than the fat and oil: less than 3.0 wt%), and an alternative cheese (also called plant cheese; solidified products of alternative milk fermented foods). Examples of the plant protein food of the present invention include a plant protein beverage (for example, alternative milk (also called plant milk)), bean curd, a meat alternative prepared from a plant raw material, alternative dairy products prepared from plant raw materials (for example, plant milk processed products such as an alternative milk fermented food), and those to which at least a flavor improved by the production method of the present invention is imparted, preferably those to which an improved property (particularly, smoothness) is imparted in addition to the improved flavor can also be exemplified. Examples of the alternative milk fermented food are as described above. Among the above examples, a preferable plant protein food of the present invention is a lactic acid fermented plant food, and among the lactic acid fermented plant foods, an alternative milk fermented food is preferable and alternative yogurt (plant yogurt) is particularly preferable.

3. Protein Improver

The present invention also provides a protein improver that can be used to improve the plant protein. The protein improver of the present invention is typically used in the production method of the present invention. The protein improver of the present invention contains a lipase and a protein deamidase as active ingredients. The details of the lipase and the protein deamidase are as described above (the section of "1. Method for Producing Plant Protein Food"), and thus description thereof is omitted.

The protein improver of the present invention can impart a distinct fermentation smell to a plant fermented food to be obtained, particularly in the case of producing a plant fermented food. Therefore, in the production of a food alternative to an animal fermented food (preferably an animal fermented food subjected to lactic acid fermentation, particularly yogurt) (that is, a plant fermented food, preferably a plant fermented food subjected to lactic acid fermentation, particularly plant yogurt), a flavor closer to that of the animal fermented food can be imparted to the plant fermented food. Therefore, the protein improver of the present invention is particularly useful when used as a fermentation smell enhancer for a plant fermented food.

The protein improver of the present invention can impart smoothness to a plant protein food to be obtained in some cases. Specific forms of imparting smoothness include forms that enhance softness, homogeneity, and/or viscosity. Examples of preferable cases where the protein improver of the present invention imparts the smoothness include cases where the origins of the protein contained in the plant protein raw material on which the protein improver of the present invention acts are beans and nuts listed in the above section 1-2, and more preferable examples thereof include cases where the origins of the protein are soybeans and coconuts. Examples of preferable cases where the protein improver of the present invention imparts the smoothness also include cases where a plant protein food obtained by using the protein improver of the present invention is a plant fermented food, more preferably a plant fermented food subjected to lactic acid fermentation, and particularly plant yogurt. Therefore, the protein improver of the present invention is also useful when used as a smoothness improver for a plant protein food.

Hereinafter, the present invention will be further described by means of Examples.

EXAMPLES

In test examples below, the hydrolysis activity of the lipase was measured by the following method.

<Activity Measurement Method (Kit Method)>

The lipase activity measurement (kit method) was performed using Lipase Kit S (manufactured by SB Bioscience Co., Ltd.) according to the manual attached to the kit. However, an attached buffer solution adjusted to pH 7 was used as a buffer solution, and acetone was used as a reaction stop solution. An activity value was calculated utilizing a calibration curve prepared using Lipase AY "Amano" 30SD (30000 u/g).

Meanwhile, the enzyme activity of the protein glutaminase was measured by the method described below using Z-Gln-Gly as a substrate.

<Activity Measurement Method>

To 100 $\mu$l of a 176 mmol/l phosphate buffer solution (pH 6.5) containing 10 mmol/l of Z-Gln-Gly, 10 $\mu$l of an enzyme solution was added, the mixture was incubated at 37°C for 60 minutes, and then 100 $\mu$l of a 12% trichloroacetic acid solution was added to stop the reaction. After centrifugation (15000 rpm, 4°C, 5 minutes), the supernatant was measured using F-kit ammonia (manufactured by Boehringer Mannheim GmbH) as follows to obtain a measured value (A1). Separately, measurement was performed in the same manner using water instead of the enzyme solution to obtain a measured value (A2). To 100 $\mu$l of F-kit ammonia reagent 2, 10 $\mu$l of the supernatant and 190 $\mu$l of water were added, the mixture was left to stand at room temperature for 5 minutes, and then an absorbance (E1) at 340 nm was measured using 100 $\mu$l of the mixture. After 1.0 $\mu$l of reagent 3 (glutamate dehydrogenase) was added to the remaining 200 $\mu$l of the mixture, the mixture was further left to stand at room temperature for 20 minutes, and then an absorbance (E2) at 340 nm of the remaining 200 $\mu$l of the mixture was measured. The amount of the enzyme that releases 1 $\mu$mol of ammonia per minute under the above conditions was defined as 1 unit (1 u), and the amount thereof was determined according to the following formula.

$$u/ml = 1.76 \times [A1(E1 - E2) - A2(E1 - E2)]$$

In the following test examples, flavor was evaluated based on the following criteria. In addition to the flavor, the property was evaluated based on the following criteria.

<Evaluation of Effect of Improving Flavor 1 (Smell)>

----: No fermentation smell, and strong raw material odor
---: No fermentation smell, and weak raw material odor
--: No fermentation smell
-: Weak fermentation smell
+: Slightly strong fermentation smell
++: Strong fermentation smell

<Evaluation of Effect of Improving Flavor 2 (Smell)>

-: Weak sour taste
+: Slightly strong sour taste
++: Strong sour taste

<Evaluation of Effect of Improving Property 1 (Smoothness)>

✕: Not smooth (hard and heterogeneous)
△: Smooth (soft and homogeneous)
○: Remarkably smooth (softness and homogeneity are remarkable)

<Evaluation of Effect of Improving Property 2 (Smoothness)>

✕: Not smooth (less viscous)
△: Slightly smooth (slightly viscous)
○: Smooth (sufficiently viscous)

<Test Example 1>

In 400 mL of water, 200 g of soybeans was immersed and then drained, and the skin was peeled. To the skin-peeled soybeans, 500 mL or water was added and stirred with a mixer for 1 minute to be ground, and the ground product was filtered to obtain soy milk. To 150 mL of the obtained soy milk (protein concentration: 10 wt%, fat-and-oil concentration: 7.5 wt%), 75 u of protein glutaminase (Protein glutaminase "Amano" 500, manufactured by Amano Enzyme Inc.) (5 u per 1 g of raw material protein) and 3400 u (kit method) of a lipase (Lipase AY "Amano" 30SD, manufactured by Amano Enzyme Inc.) (300 u per 1 g of raw material fat and oil) were added and treated at 50°C for 1 hour. The resulting product was thermally treated at 72°C for 15 minutes to deactivate the enzyme, and then cooled to 40°C. To 100 mL of the obtained enzyme-treated soy milk, 3 g of sugar and 15 g of commercially available soy milk yogurt (Rivon Soygurt, manufactured by The Thai Dairy Industry Co., Ltd.) as a starter were added, fermented at 42°C for 10 hours, and then cooled to 4°C, thereby obtaining soy milk yogurt (Example 1). For comparison, soy milk yogurt was obtained by the same method as in Example 1, except that only protein glutaminase was used in the enzyme treatment (Comparative Example 2). As a control group, soy milk yogurt was obtained by the same method as in Example 1, except that the enzyme treatment was omitted (Comparative Example 1).
The obtained soy milk yogurt was subjected to a sensory test for the flavor 1 and the property 1 described above. The results of the sensory test are shown in Table 1.

[Table 1]

| | PG addition amount (u/protein 1 g) | Lipase addition amount (u/fat and oil 1 g) | Flavor 1 (smell) | Property 1 (smoothness) |
|---|---|---|---|---|
| Comparative Example 1 | 0 | 0 | ---- | × |
| Comparative Example 2 | 5 | 0 | --- | △ |
| Example 1 | 5 | 300 | ++ | ○ |

As shown in Table 1, the soy milk yogurt without the enzyme treatment (Comparative Example 1) had a strong soybean odor, was hard and heterogeneous, and thus had no smoothness. Although the soybean odor was reduced by the PG treatment and a smooth texture was obtained (Comparative Example 2), the effect of improving these properties was insufficient. On the other hand, it was surprisingly found that, by addition of the lipase treatment to the PG treatment (that is, the use of PG and the lipase in combination, the fermentation smell was changed to a strong fermentation smell like normal yogurt and smoothness was also further improved (Example 1).

<Test Example 2>

To 400 mL of water, 200 g of soybeans was immersed. To the soybeans (with skin) after drainage, 500 mL of water was added and stirred with a mixer for 1 minute to be ground, and the ground product was filtered to obtain soy milk. To 150 mL of the soy milk (protein concentration: 10 wt%, fat-and-oil concentration: 7.5 wt%), 75 u or 225 u of protein glutaminase (Protein glutaminase "Amano" 500, manufactured by Amano Enzyme Inc.) (5 u or 15 u per 1 g of raw material protein) and 170 u (kit method) of Lipase AY (Lipase AY "Amano" 30SD, manufactured by Amano Enzyme Inc.) (15 u per 1 g of raw material fat and oil) were added and treated at 50°C for 1 hour. The resulting product was thermally treated at 72°C for 15 minutes to deactivate the enzyme, and then cooled to 40°C. To 100 mL of the obtained enzyme-treated soy milk, 3 g of sugar and 15 g of commercially available soy milk yogurt (Rivon Soygurt, manufactured by The Thai Dairy Industry Co., Ltd.) as a starter were added, fermented at 42°C for 10 hours, and then cooled to 4°C, thereby obtaining soy milk yogurt (Example 2). For comparison, soy milk yogurt was obtained by the same method as in Example 2, except that only protein glutaminase was used in the enzyme treatment (Comparative Examples 3 and 4). As a control group, soy milk yogurt was obtained by the same method as in Example 2, except that the enzyme treatment was omitted (Comparative Example 5).

The obtained soy milk yogurt was subjected to a sensory test for the flavor 1 and the property 1 described above. The results of the sensory test are shown in Table 2.

[Table 2]

| | PG addition amount (u/protein 1 g) | Lipase addition amount (u/fat and oil 1 g) | Flavor 1 (smell) | Property 1 (smoothness) |
|---|---|---|---|---|
| Comparative Example 5 | 0 | 0 | ---- | × |
| Comparative Example 3 | 5 | 0 | --- | △ |
| Comparative Example 4 | 15 | 0 | --- | △ |
| Example 2 | 15 | 15 | ++ | ○ |

As shown in Table 2, even when whole soybeans (soybeans with skin) were used, a strong fermentation smell preferable as yogurt could be imparted instead of a soybean odor by using PG and the lipase in combination, and a smooth texture was obtained (Example 2). On the other hand, only by the PG treatment, the soybean odor was slightly reduced, and the texture was soft and smooth, but the improving effect thereof was insufficient (Comparative Examples 3 and 4).

<Test Example 3>

To 400 mL of water, 200 g of almonds was immersed. To the almonds after drainage, 500 mL of water was added and stirred with a mixer for 1 minute to be ground, and the ground product was filtered to obtain almond milk. To 150 mL of the almond milk (protein concentration: 6 wt%, fat-and-oil concentration: 14 wt%), 45 u of protein glutaminase (Protein glutaminase "Amano" 500, manufactured by Amano Enzyme Inc.) (5 u per 1 g of raw material protein) and 3400 u (kit method) of Lipase AY (Lipase AY "Amano" 30SD, manufactured by Amano Enzyme Inc.) (160 u per 1 g of raw material fat and oil) were added and treated at 50°C for 1 hour. The resulting product was thermally treated at 72°C for 15 minutes to deactivate the enzyme, and then cooled to 40°C. To 100 mL of the obtained enzyme-treated almond milk, 3 g of sugar and 15 g of commercially available almond yogurt (Hooray almond your-gurt, manufactured by Crossmax Retail Co., Ltd.) as a starter were added, fermented at 42°C for 10 hours, and then cooled to 4°C, thereby obtaining almond yogurt (Example 3). For comparison, almond yogurt was obtained by the same method as in Example 3, except that only protein glutaminase was used in the enzyme treatment (Comparative Example 6). Almond yogurt was obtained by the same method as in Example 3, except that the enzyme treatment was omitted (Comparative Example 7).

The obtained almond yogurt was subjected to a sensory test for the flavor 1 and the flavor 2 described above. The results of the sensory test are shown in Table 3.

[Table 3]

|  | PG addition amount (u/protein 1 g) | Lipase addition amount (u/fat and oil 1 g) | Flavor 1 (smell) | Flavor 2 (taste) |
|---|---|---|---|---|
| Comparative Example 7 | 0 | 0 | -- | - |
| Comparative Example 6 | 5 | 0 | - | - |
| Example 3 | 5 | 160 | ++ | ++ |

As shown in Table 3, although a slight improving effect on the smell only by the PG treatment was recognized also in the almond yogurt, the degree thereof was insufficient (Comparative Example 6), but it was found that the fermentation smell can be intensified by further concurrently using the lipase treatment (Example 3). Regarding the taste, although the effect that the sour taste can be enhanced only by the PG treatment could not be confirmed (Comparative Example 6), it was found that a strong sour taste like yogurt can be imparted by further concurrently using the lipase treatment (Example 3).

<Test Example 4>

To 400 mL of water, 200 g of oats was immersed. To the oats after drainage, 500 mL of water was added and stirred with a mixer for 1 minute to be ground, and the ground product was filtered to obtain oat milk. To 150 mL of the oat milk (protein concentration: 3 wt%, fat-and-oil concentration: 2.9 wt%), 23 u of protein glutaminase (Protein glutaminase "Amano" 500, manufactured by Amano Enzyme Inc.) (5 u per 1 g of raw material protein) and 1300 u (kit method) of Lipase AY (Lipase AY "Amano" 30SD, manufactured by Amano Enzyme Inc.) (300 u per 1 g of raw material fat and oil) were added and treated at 50°C for 1 hour. The resulting product was thermally treated at 72°C for 15 minutes to deactivate the enzyme, and then cooled to 40°C. To 100 mL of the obtained enzyme-treated oat milk, 3 g of sugar and 15 g of commercially available almond yogurt (Hooray almond your-gurt, manufactured by Crossmax Retail Co., Ltd.) as a starter were added, fermented at 42°C for 10 hours, and then cooled to 4°C, thereby obtaining oat yogurt (Example 4). For comparison, oat yogurt was obtained by the same method as in Example 4, except that only protein glutaminase was used in the enzyme treatment (Comparative Example 8). Oat yogurt was obtained by the same method as in Example 4, except that the enzyme treatment was omitted (Comparative Example 9).

The obtained oat yogurt was subjected to a sensory test for the flavor 1 described above. The results of the sensory test are shown in Table 4.

[Table 4]

|  | PG addition amount (u/protein 1 g) | Lipase addition amount (u/fat and oil 1 g) | Flavor 1 (smell) |
|---|---|---|---|
| Comparative Example 9 | 0 | 0 | ---- |

(continued)

|  | PG addition amount (u/protein 1 g) | Lipase addition amount (u/fat and oil 1 g) | Flavor 1 (smell) |
|---|---|---|---|
| Comparative Example 8 | 5 | 0 | - |
| Example 4 | 5 | 300 | ++ |

As shown in Table 4, although the effect of improving flavor only by the PG treatment was recognized also in the oat yogurt, the degree thereof was insufficient (Comparative Example 8), but it was found that a strong fermentation smell preferable as yogurt can be imparted by further concurrently using the lipase treatment (Example 4).

<Test Example 5>

To 100 mL of water, 50 g of coconut milk was added to obtain diluted coconut milk (liquid). To 150 mL of the obtained diluted coconut milk (protein concentration: 1 wt%, fat-and-oil concentration: 6 wt%), 22 u of protein glutaminase (Protein glutaminase "Amano" 500, manufactured by Amano Enzyme Inc.) (15 u per 1 g of raw material protein) and 27 u (kit method) of Lipase AY (Lipase AY "Amano" 30SD, manufactured by Amano Enzyme Inc.) (3 u per 1 g of raw material fat and oil) were added and treated at 50°C for 1 hour. 3 g of processed starch and 4.5 g of sugar were added, and the resulting product was thermally treated at 85°C for 30 minutes to deactivate the enzyme, and then cooled to 40°C. To 100 mL of the obtained enzyme-treated coconut milk, 15 g of commercially available coconut yogurt (Agrilife Cocogurt, manufactured by Earth Born Co., Ltd.) was added, fermented at 42°C for 10 hours, and then cooled to 4°C, thereby obtaining coconut yogurt (Example 5). For comparison, coconut yogurt was obtained by the same method as in Example 5, except that only protein glutaminase was used in the enzyme treatment (Comparative Example 10), and coconut yogurt was obtained by the same method as in Example 5, except that the enzyme treatment was omitted (Comparative Example 11).
The obtained coconut yogurt was subjected to a sensory test for the flavor 1 and the property 2 described above. The results of the sensory test are shown in Table 5.

[Table 5]

|  | PG addition amount (u/protein 1 g) | Lipase addition amount (u/fat and oil 1 g) | Flavor 1 (smell) | Property 2 (smoothness) |
|---|---|---|---|---|
| Comparative Example 11 | 0 | 0 | ---- | × |
| Comparative Example 10 | 15 | 0 | ---- | ○ |
| Example 5 | 15 | 3 | ++ | ○ |

As shown in Table 5, also in the coconut yogurt, it was found that, by using PG and the lipase in combination, the smell of the coconut yogurt could be modified into a strong fermentation smell preferable as yogurt, and additionally, viscosity was imparted to the coconut yogurt, so that a smooth texture was obtained (Example 5). On the other hand, a smooth texture was obtained only by the PG treatment, but the coconut smell was not changed, and the fermentation smell could not be imparted at all (Comparative Example 10).

INDUSTRIAL APPLICABILITY

According to the production method of the present invention, a plant protein food having excellent flavor can be obtained. Examples of the plant protein food produced by the present invention include plant fermented foods (for example, a lactic acid fermented plant food, more specifically, an alternative milk fermented food, further specifically, alternative yogurt (plant yogurt), an alternative dairy product lactobacillus beverage, an alternative lactobacillus beverage, an alternative cheese (plant cheese), and also include a plant protein beverage (for example, alternative milk (plant milk)), bean curd, a meat alternative prepared from a plant raw material, and alternative dairy products prepared from plant raw materials (for example, plant milk processed products such as an alternative milk fermented food).
This invention is not limited at all by the description of the above-described embodiments for carrying out the invention and Examples. Various modifications are also encompassed in this invention within the scope that does not deviate from the descriptions in the claims and can be easily conceived by persons skilled in the art. The whole contents of the articles, patent publications, patents, and the like which are clearly indicated in the present specification are incorporated herein by reference.

**Claims**

1. A method for producing a plant protein food, comprising causing a lipase and a protein deamidase to act on a plant protein raw material containing a protein and a fat and oil.

2. The method for producing a plant protein food according to claim 1, wherein the plant protein raw material is a soybean-derived raw material, an oat-derived raw material, an almond-derived raw material, or a coconut-derived raw material.

3. The method for producing a plant protein food according to claim 1 or 2, wherein the method comprises steps below:

    (1) a step of preparing a plant protein raw material containing a protein and a fat and oil; and
    (2) a step of treating the prepared plant protein raw material with a lipase and a protein deamidase.

4. The method for producing a plant protein food according to claim 3, wherein the plant protein food is a fermented food, and the method comprises a step below after the step (2):
   (3) a step of fermenting with a microorganism.

5. The method for producing a plant protein food according to claim 4, wherein the fermented food is a lactic acid fermented food, and the microorganism in the step (3) is lactobacillus.

6. The method for producing a plant protein food according to any one of claims 1 to 5, wherein the lipase is a Candida cylindracea-derived lipase.

7. The method for producing a plant protein food according to any one of claims 1 to 6, wherein the protein deamidase is an enzyme acting on a glutamine residue in a protein.

8. The method for producing a plant protein food according to claim 7, wherein the protein deamidase is protein glutaminase.

9. A plant protein improver comprising a lipase and a protein deamidase.

10. The plant protein improver according to claim 9, wherein the lipase is a Candida cylindracea-derived lipase.

11. The plant protein improver according to claim 9 or 10, wherein the protein deamidase is an enzyme acting on a glutamine residue in a protein.

12. The plant protein improver according to claim 11, wherein the protein deamidase is protein glutaminase.

13. The plant protein improver according to any one of claims 9 to 11, wherein the plant protein improver is used as a fermentation smell enhancer for a plant fermented food.

14. The plant protein improver according to any one of claims 9 to 12, wherein the plant protein improver is used as a smoothness improver for a plant protein food.

## INTERNATIONAL SEARCH REPORT

| International application No. |
| --- |
| PCT/JP2021/026185 |

### A. CLASSIFICATION OF SUBJECT MATTER

```
Int. Cl. C12P1/00(2006.01)i, A23L5/00(2016.01)i, A23L29/00(2016.01)i, A23L33/185(2016.01)i,
C12N9/20(2006.01)n, A23C11/10(2021.01)i, A23L11/60(2021.01)i, A23L11/65(2021.01)i, A23L11/00(2021.01)i
FI: A23L33/185, A23C11/10, A23L5/00 J, A23L5/00 M, A23L11/00 Z, A23L29/00, C12P1/00 A, C12N9/20
```

According to International Patent Classification (IPC) or to both national classification and IPC

### B. FIELDS SEARCHED

Minimum documentation searched (classification system followed by classification symbols)

```
Int. Cl. C12P1/00, A23L5/00, A23L29/00, A23L33/185, C12N9/20, A23C11/10,
A23L11/60, A23L11/65, A23L11/00
```

Documentation searched other than minimum documentation to the extent that such documents are included in the fields searched

```
Published examined utility model applications of Japan     1922-1996
Published unexamined utility model applications of Japan   1971-2021
Registered utility model specifications of Japan           1996-2021
Published registered utility model applications of Japan   1994-2021
```

Electronic data base consulted during the international search (name of data base and, where practicable, search terms used)

```
JSTPlus/JMEDPlus/JST7580 (JDreamIII); CAplus/MEDLINE/EMBASE/BIOSIS
(STN);AGRICOLA (STN);FSTA (STN)
```

### C. DOCUMENTS CONSIDERED TO BE RELEVANT

| Category* | Citation of document, with indication, where appropriate, of the relevant passages | Relevant to claim No. |
| --- | --- | --- |
| X | JP 2019-097570 A (AJINOMOTO CO., INC.) 24 June 2019 (2019-06-24), claims, paragraph [0018], examples | 1-3, 6-7, 9-11, 14 |
| X | JP 2020-099295 A (AJINOMOTO CO., INC.) 02 July 2020 (2020-07-02), claims, paragraph [0014], examples | 1, 3, 6-7, 9-11, 14 |
| X | JP 2000-050887 A (AMANO PHARMA CO., LTD.) 22 February 2000 (2000-02-22), claims, paragraph [0060], examples | 1-4, 7-9, 11-12, 14 |
| X | JP 2016-502868 A (IMPOSSIBLE FOODS INC.) 01 February 2016 (2016-02-01), claims, paragraphs [0158]-[0160], examples | 1-7, 9-11, 13-14 |
| Y | | 1-14 |

☒ Further documents are listed in the continuation of Box C.  ☒ See patent family annex.

| | |
| --- | --- |
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 07.09.2021 | 14.09.2021 |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.

PCT/JP2021/026185

C (Continuation).   DOCUMENTS CONSIDERED TO BE RELEVANT

| Category* | Citation of document, with indication, where appropriate, of the relevant passages | Relevant to claim No. |
|---|---|---|
| Y | WO 2009/113628 A1 (AJINOMOTO CO., INC.) 17 September 2009 (2009-09-17), claims, examples | 1-14 |

Form PCT/ISA/210 (continuation of second sheet) (January 2015)

**INTERNATIONAL SEARCH REPORT**
Information on patent family members

International application No.

PCT/JP2021/026185

| Patent Documents referred to in the Report | Publication Date | Patent Family | Publication Date |
|---|---|---|---|
| JP 2019-097570 A | 24.06.2019 | (Family: none) | |
| JP 2020-099295 A | 02.07.2020 | (Family: none) | |
| JP 2000-050887 A | 22.02.2000 | US 2004/0072318 A1 claims, paragraph [0092], examples | |
| JP 2016-502868 A | 01.02.2016 | WO 2014/110540 A1 claims, pp. 56, 57, examples US 2015/0305361 A1 EP 2943077 A1 | |
| WO 2009/113628 A1 | 17.09.2009 | US 2011/0064847 A1 claims, examples EP 2267146 A1 | |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H06030710 A
- JP 2011525356 A
- JP H06197688 A
- WO 2006075772 A
- JP 2003250482 A
- JP H07031371 A
- WO 2015133590 A

**Non-patent literature cited in the description**

- *Eur J Biochem,* 2001, vol. 268 (5), 1410
- **S YAMAGUCHI 1 ; D J JEENES ; D B ARCHER.** Protein-glutaminase From Chryseobacterium Proteolyticum, an Enzyme That Deamidates Glutaminyl Residues in Proteins. Purification, Characterization and Gene Cloning. *Front Microbiol,* 2018, vol. 9, 1975
- Molecular Approaches to Improving Food Quality and Safety. Van Nostrand Reinhold, 1992, 37